(19) Europäisches Patentamt
European Patent Office
Office européen des brevets

(11) Veröffentlichungsnummer: **0 251 118 B1**

# (12) EUROPÄISCHE PATENTSCHRIFT

(45) Veröffentlichungstag der Patentschrift: **11.04.90**

(51) Int. Cl.⁴: **C07C 231/08**, C07C 233/17

(21) Anmeldenummer: **87108949.6**

(22) Anmeldetag: **23.06.87**

## (54) Verfahren zur Herstellung von N-substituierten Formamiden.

(30) Priorität: **01.07.86 DE 3622013**

(43) Veröffentlichungstag der Anmeldung: **07.01.88 Patentblatt 88/1**

(45) Bekanntmachung des Hinweises auf die Patenterteilung: **11.04.90 Patentblatt 90/15**

(84) Benannte Vertragsstaaten: **DE FR GB**

(56) Entgegenhaltungen:
**DE-A- 3 520 829**
**FR-A- 2 558 156**

**PATENT ABSTRACTS OF JAPAN, Band 10, Nr. 276 (C-373)[2332], 19. September 1986; & JP - A - 61 97309**

(73) Patentinhaber: **BASF Aktiengesellschaft, Carl-Bosch-Strasse 38, D-6700 Ludwigshafen(DE)**

(72) Erfinder: **Fikentscher, Rolf, Dr., Von-Stephan-Strasse 27, D-6700 Ludwigshafen(DE)**
Erfinder: **Kroener, Michael, Dr., Eislebener Weg 8, D-6800 Mannheim 31(DE)**
Erfinder: **Oftring, Alfred, Dr., Berner Weg 26, D-6700 Ludwigshafen(DE)**

EP 0 251 118 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Aus der US-PS 4,567,300 ist ein Verfahren zur Herstellung von N-substituierten Formamiden der Formel

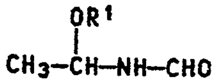 (I)

bekannt, in der $R^1$ ein Wasserstoffatom oder den Rest eines primären oder sekundären Alkohols bedeutet.

Die Verbindungen der Formel (I) werden erhalten, indem man Formamid mit Acetaldehyd in Gegenwart eines basischen Katalysators, z.B. Kaliumcarbonat, bei Temperaturen von -10 bis 100, vorzugsweise 0 bis 40°C umsetzt. Das dabei entstehende N-(α-Hydroxyethyl)formamid wird bei der Umsetzung in kristalliner Form abgeschieden. Führt man die Umsetzung in Abwesenheit eines Verdünnungsmittels durch, so ergeben sich Schwierigkeiten bei der Handhabung des Produktes. Die Umsetzung von Formamid und Acetaldehyd wird daher vorzugsweise in einem inerten Verdünnungsmittel, z.B. aliphatischen oder aromatischen Kohlenwasserstoffen vorgenommen. Vorzugsweise verwendet man n-Hexan, legt darin Formamid und Kaliumcarbonat vor und leitet bei einer Temperatur von 25°C Acetaldehyd gasförmig ein. Durch das Einleiten des Acetaldehyds in gasförmigem Zustand erhält man relativ pro Volumeneinheit eine schwächere exotherme Reaktion im Vergleich zu einer Zugabe des Acetaldehyds in flüssigem Zustand. Um das N-(α-Hydroxyethyl)formamid unter den Reaktionsbedingungen zur Kristallisation zu bringen, ist es erforderlich, Impfkristalle zuzusetzen. Nach Beendigung der Umsetzung von Formamid und Acetaldehyd muß das Reaktionsprodukt vom Suspensionsmittel abgetrennt werden. In der folgenden Stufe wird das N-(α-Hydroxyethyl)formamid mit überschüssigem Alkohol versetzt und in Gegenwart eines sauren Katalysators verethert. Die dabei erhältlichen Verbindungen der Formel (I), in denen $R^1$ eine von Wasserstoff unterschiedliche Bedeutung hat, sind Ausgangsprodukte für die Herstellung von N-Vinylformamid, aus dem Polymerisate mit besonders interessanten Eigenschaften erhältlich sind.

Das oben beschriebene Verfahren hat mehrere Nachteile. So benötigt man beispielsweise eine relativ große Menge an inertem Lösemittel, das nach beendeter Reaktion vom auskristallisierten N-(α-Hydroxyethyl)formamid abgetrennt werden muß. Diese Prozedur ist aufgrund der thermischen Labilität des Produkts zumindest im großtechnischen Maßstab mit erheblichen Schwierigkeiten verbunden. Trennt man das inerte Lösemittel nicht ab, so erhält man bei der anschließenden Veretherung geringere Ausbeuten als bei Einsatz des reinen kristallinen Produkts. Außerdem ist während der Reaktion der Zusatz von Impfkristallen unerläßlich. Vor der Kristallisation liegt das N-(α-Hydroxyethyl)formamid in einer sogenannten unterkühlten Schmelze in einem übersättigten Zustand vor. Der Zusatz der Impfkristalle bewirkt eine spontane Kristallisation, bei der es häufig zu Klumpenbildung kommt, wodurch die freiwerdende Kristallisationswärme nur sehr langsam abgeführt werden kann.

Der vorliegenden Erfinding liegt die Aufgabe zugrunde, ein Verfahren zur Herstellung von N-substituierten Formamiden der Formel

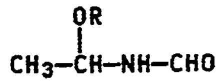 (II),

in der R=$C_1$- bis $C_8$-Alkyl bedeutet, zur Verfügung zu stellen, bei dem man die durch Basen katalysierte Reaktion von Formamid mit Aceteldehyd in Abwesenheit von größeren Mengen an inertem Suspensionsmittel auch in großtechnischem Maßstab so durchführen kann, daß das N-(α-Hydroxyethyl)formamid ohne Klumpenbildung auskristallisiert.

Die Aufgabe wird erfindungsgemäß mit einem Verfahren zur Herstellung von N-substituierten Formamiden der Formel

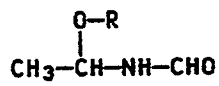 ,

in der R=$C_1$- bis $C_8$-Alkyl bedeutet, durch Reaktion von Formamid mit Acetaldehyd in Gegenwart eines basischen Katalysators zu N-(α-Hydroxyethyl)formamid und anschließende Veretherung des N-(α-Hydroxyethyl)formamids mit $C_1$- bis $C_8$-gesättigten Alkoholen in Gegenwart von sauren Katalysatoren gelöst, wenn man die Reaktion von Formamid mit Acetaldehyd zusätzlich in Gegenwart eines $C_1$- bis $C_8$-Alkohols durchführt, wobei das Gewichtsverhältnis Formamid zu Alkohol 1:1 bis 15:1 beträgt. Dadurch erübrigt sich der Einsatz von aliphatischen oder aromatischen Kohlenwasserstoffen als Verdünnungsmittel, so daß eine Abtrennung der Verdünnungsmittel vor der Veretherung entfällt. Außerdem fällt das N-(α-Hydroxyethyl)formamid in feinkristalliner Form ohne Bildung von Klumpen an und kann ohne Reinigungsoperationen direkt nach weiterer Zugabe von Alkohol verethert werden. Ein Zusatz von Impfkristallen ist nicht erforderlich.

Die Verbindungen der Formel (II) werden durch Reaktion von Formamid mit Acetaldehyd unter den in der US-PS 4,567,300 beschriebenen Bedingungen erhalten, wobei man zum Unterschied zum bekannten Verfahren anstelle einer größeren Menge eines inerten Verdünnungsmittels eine geringe Menge eines $C_1$- bis $C_8$-gesättigten Alkohols oder eine Mischung solcher Alkohole in der ersten Stufe der Reaktion einsetzt. Erstaunlicherweise stört der Zusatz des Alkohols bei der Ethylolierungsreaktion nicht, obwohl dadurch teilweise das entsprechende Halbacetal des Acetaldehyds gebildet wird. Das Molverhältnis von Formamid zu Acetaldehyd zur Herstellung von N-(α-Hydroxyethyl)formamid beträgt 1:1 bis 1:5, vorzugsweise 1:1 bis 1:1,5. Der Acetaldehyd wird gasförmig oder in flüssiger Form in die Mischung aus Formamid, Alkohol und basichem Katalysator eingebracht. Die Reaktionstemperatur beträgt -10 bis 60°C, vorzugsweise 0 bis 40°C. Da der Schmelzpunkt des N-(α-Hydroxyethyl)formamids in dem Bereich von 52,5 bis 53,8°C liegt, wird die Umsetzung von Formamid und Acetaldehyd vorzugsweise unterhalb des Schmelzpunkts des N-(α-Hydroxyethyl)formamids durchgeführt.

Die Reaktion von Formamid mit Acetaldehyd erfolgt in Gegenwart von basischen Katalysatoren. Geeignete basische Katalysatoren sind beispielsweise die Oxide, Hydroxide und Carbonate von Alkalimetallen und Erdalkalimetallen sowie schwach basische Salze, die sich von einer starken Base und einer schwachen Säure ableiten, z.B. Natriumphosphat, Kaliumpyrophosphat, Natriumpyrophosphat, Natriumacetat, Natriumphenolat und Natriumborat. Als Katalysator eignen sich außerdem tertiäre Amine und quaternäre Ammoniumverbindungen, z.B. Tetrabutylammoniumhydroxid oder Tetrabutylammoniumcarbonat sowie Ionenaustauscherharze, die basische Gruppen enthalten. Insbesondere verwendet man schwach basische Salze, die als Reaktionsprodukt einer starken Base und einer schwachen Säure aufzufassen sind und die einen $pK_S$-Wert im Bereich von 4 bis 15 bei einer Konzentration von 0,01 mol/1 einer wäßrigen Lösung bei 25°C haben. Derartige Salze sind beispielsweise die Lithium-, Natrium- oder Kaliumsalze von organischen Carbonsäuren, z.B. Ameisensäure, Essigsäure, Propionsäure und Stearinsäure. Besonders bevorzugte basische Katalysatoren sind Kaliumcarbonat, Natriumcarbonat, Kaliumphosphat, Natriumphosphat, Kaliumpyrophosphat und Natriumpyrophosphat. Bezogen auf Formamid verwendet man 0,01 bis 10, vorzugsweise 0,1 bis 5 mol-% eines basischen Katalysators oder einer Mischung von basischen Katalysatoren.

Um die Umsetzung von Formamid mit Acetaldehyd in Abwesenheit größerer Mengen eines inerten Lösemittels und unter Vermeidung des Zusatzes von Impfkristallen durchführen zu können, wird die Reaktion von Formamid mit Acetaldehyd in Gegenwart eines $C_1$- bis $C_8$-Alkohols durchgeführt. Man benötigt nur eine relativ geringe Menge eines Alkohols. Die Alkoholmenge ist begrenzt durch die Löslichkeit des N-(α-Hydroxyethyl)formamids im Alkohol.

Das Gewichtsverhältnis von Formamid zu einem $C_1$- bis $C_8$-Alkohol beträgt 1:1 bis 15:1, vorzugsweise 5:1 bis 12:1. Geeignete Alkohole sind beispielsweise Methanol, Ethanol, n-Propanol, Isopropanol, n-Butanol, Isobutanol, tert.-Butanol, n-Pentanol, Isopentanol, n-Hexanol, Cyclohexanol sowie n-Octyl- und Isooctylalkohol. Vorzugsweise führt man die Umsetzung von Formamid mit Acetaldehyd in Gegenwart eines $C_1$- bis $C_4$-Alkohols durch. Aus dieser Gruppe von Alkoholen ist Methanol besonders bevorzugt. Das erfindungsgemäße Verfahren wird vorzugsweise so durchgeführt, daß man Formamid und die erforderliche Menge an Alkohol sowie den basischen Katalysator vorlegt, dann in die Mischung Acetaldehyd gasförmig einleitet oder in flüssiger Form zugibt. Das dabei in kristalliner Form anfallende N-(α-Hydroxyethyl)formamid kann ohne Reinigungsoperation durch Zugabe eines geeigneten Alkohols und Erniedrigen des pH-Wertes der Reaktionsmichung verethert werden.

Geeignete Alkohole, die für die Veretherung in Betracht kommen, sind die bereits oben genannten einwertigen $C_1$- bis $C_8$-Alkohole sowie diejenigen, die außerdem in der US-PS 4,567,300 zur Herstellung der Ether angegeben sind, nämlich 2-Methoxyethanol, 2-Ethoxyethanol, 2-Propoxyethanol, 2-Butoxyethanol, Diethylenglykol, Diethylenglykolmonomethylether, Ethylenglykol, Propylenglykol, 1,4-Butandiol und Diethylenglykol. Vorzugsweise verwendet man einwertige primäre Alkohole mit 1 bis 4 Kohlenstoffatomen. Von dieser Gruppe wird besonders bevorzugt Methanol eingesetzt. Bezogen auf 1 mol N-(α-Hydroxyethyl)formamid setzt man bei der Veretherung 1 bis 20, vorzugsweise 1 bis 6 mol eines Alkohols oder eines Alkoholgemisches ein. Die Veretherung wird in Gegenwart von sauren Katalysatoren durchgeführt. Hierfür kommen ebenfalls alle diejenigen sauren Katalysatoren in Betracht, die in der US-PS 4,567,300 angegeben sind, z.B. Mineralsäuren, Carbonsäuren, Ionenaustauscher mit sauren Gruppen sowie andere feste saure Katalysatoren. Geeignete Katalysatoren sind beispielsweise Schwefelsäure, Salzsäure, Bromwasserstoffsäure, Amidosulfonsäure, Methansulfonsäure, Ethansulfonsäure, p-Toluolsulfonsäure, vernetzte Polystyrolsulfonsäuren und Phosphorsäure. Bezogen auf N-(α-Hydroxyethyl)formamid benötigt man 0,1 bis 10, vorzugsweise 1 bis 5 mol-% eines sauren Katalysators. Die Reaktionstemperatur bei der Veretherung beträgt -20 bis +80, vorzugsweise -10 bis +60°C. Die Ether der Formel (II) sind bei 20°C flüssige Verbindungen, die unter vermindertem Druck destilliert und so in reiner Form hergestellt werden können. Die Verbindungen der Formel (II) werden als Ausgangsprodukt zur Herstellung von N-Vinylformamid verwendet. Das Monomere erhält man, indem man die Verbindung der Formel (II) bei Temperaturen oberhalb von 400°C und Drücken von 10 bis 200 mbar pyrolisiert. Dabei wird unter Abspaltung von Alkohol N-Vinylformamid gebildet. Aus N-Vinylformamid können z.B. gemäß der US-PS 4,421,602 Polymerisate hergestellt werden, die gegenüber den nächstvergleichbaren Polymerisaten des Standes der Technik bei der Herstellung von Papier erhöhte Entwässerungsgeschwindigkeit und Retention aufweisen sowie Polymerisate, die gemäß der US-PS 4,444,667 besonders wirksame Flockungsmittel für Schlämme darstellen.

Beispiel 1

In einem 700 l fassenden Kessel, der mit einem Rührer und einer Kühleinrichtung ausgestattet ist, legt man 70,2 kg Formamid vor und löst darin 1,0 kg Kaliumcarbonat. Anschließend fügt man 7,0 kg Methanol (Gewichtsverhältnis Formamid : Methanol = 10 : 1) zu. Innerhalb von 5 Stunden gibt man zu der vorgelegten Lösung 50,0 kg Acetaldehyd gasförmig zu und hält die Reaktionstemperatur im Bereich von 20 bis 30°C. Die Kristallisation des N-(α-Hy-

droxyethyl)formamids beginnt ohne Zusatz von Impfkristallen, nachdem etwa zwei Drittel der für einen stöchiometrischen Umsatz erforderlichen Acetaldehydmenge zugeführt sind. Die Kristallisation ist daran zu erkennen, daß die Temperatur auf ca. 35°C ansteigt. Man kühlt das Reaktionsgemisch auf 20°C und gibt danach die restliche Acetaldehydmenge, d.h. 25,0 kg in flüssiger Form innerhalb von 1,5 Stunden zu. Nach beendeter Acetaldehydzugabe und nach abgeschlossener Kristallisation wird das Reaktionsgemisch auf 10°C abgekühlt und noch 1 Stunde bei dieser Temperatur weitergerüht. Bei einer Temperatur von 10°C setzt man dann 50 kg Methanol, 8,2 kg 50-%ige methanolische Schwefelsäure und noch 140 kg Methanol (insgesamt 190 kg) zu. Das Reaktionsgemisch wird bei einem pH-Wert von 1,5 noch 2 Stunden bei 20°C gerührt, danach mit 50-%iger wäßriger Natronlauge neutralisiert. Die anorganischen Salze werden abfiltriert und das Filtrat mit Hilfe einer Dünnfilmverdampfung bei 100°C und einem Druck von 200 mbar von leichtflüchtigen Anteilen befreit. Die Ausbeute beträgt 95%, bezogen auf eingesetztes Formamid. Der Rückstand kann entweder einer fraktionierten Destillation unterworfen werden oder direkt bei einer Temperatur von 450°C und einem Druck von 50 mbar in N-Vinylformamid und Methanol thermisch zerlegt werden.

Beispiel 2

Beispiel 1 wird mit der Ausnahme wiederholt, daß man die gesamte Menge an Acetaldehyd innerhalb von 4 Stunden kontinuierlich in flüssiger Form zum Reaktionsgemisch zuführt. Nach Zugabe von ca. 60 % der Acetaldehydmenge beginnt die Kristallisation des N-(α-Hydroxyethyl)formamids. Die Ausbeute beträgt 93 %, bezogen auf eingesetztes Formamid.

## Patentansprüche

1. Verfahren zur Herstellung von N-substituierten Formamiden der Formel

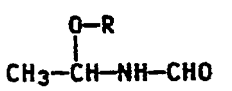,

in der R den Rest eines $C_1$- bis $C_8$-Alkohols bedeutet, durch Reaktion von Formamid mit Acetaldehyd in Gegenwart eines basischen Katalysators zu N-(α-Hydroxyethyl)formamid und anschließende Veretherung des N-(α-Hydroxyethyl)formamids mit einem $C_1$- bis $C_8$-Alkohol in Gegenwart von sauren Katalysatoren, dadurch gekennzeichnet, daß man die Reaktion von Formamid mit Acetaldehyd in Gegenwart eines $C_1$-bis $C_8$-Alkohols durchführt, wobei das Gewichtsverhältnis Formamid zu Alkohol 1:1 bis 15:1 beträgt.

2. Verfahren nach Anspurch 1, dadurch gekennzeichnet, daß man die Reaktion von Formamid mit Acetaldehyd in Gegenwart eines $C_1$-bis $C_4$-Alkohols durchführt, wobei das Gewichtsverhältnis von Formamid zu $C_1$- bis $C_4$-Alkohol 5:1 bis 12:1 beträgt.

3. Verfahren nach den Ansprüchen 1 und 2, dadurch gekennzeichnet, daß man die Reaktion von Formamid mit Acetaldehyd in Gegenwart von Methanol durchführt.

4. Verfahren nach den Ansprüchen 1 bis 3, dadurch gekennzeichnet, daß man Formamid mit Acetaldehyd in Gegenwart von Methanol und Natrium- und/oder Kaliumcarbonat umsetzt und das Reaktionsprodukt anschließend ohne vorherige Abtrennung des N-(α-Hydroxyethyl)formamids mit Methanol in Gegenwart von sauren Katalysatoren verethert.

## Claims

1. A process for the preparation of an N-substituted formamide of the formula

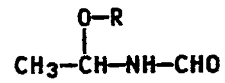

where R is a radial of a $C_1$–$C_8$-alcohol, by reacting formamide with acetaldehyde in the presence of a basic catalyst to give N-(α-hydroxyethyl)-formamide and then etherifying the N-(α-hydroxyethyl)-formamide with a $C_1$–$C_8$-alcohol in the presence of an acidic catalyst, wherein the reaction of formamide with acetaldehyde is carried out in the presence of a $C_1$–$C_8$-alcohol, the weight ration of formamide to alcohol being from 1:1 to 15:1.

2. A process as claimed in claim 1, wherein the reaction of formamide with acetaldehyde is carried out in the presence of a $C_1$–$C_4$-alcohol, the weight ratio of formamide to $C_1$–$C_4$-alcohol being from 5:1 to 12:1.

3. A process as claimed in claims 1 and 2, wherein the reaction of formamide with acetaldehyde is carried out in the presence of methanol.

4. A process as claimed in any of claims 1 to 2, wherein formamide is reacted with acetaldehyde in the presence of methanol and sodium carbonate and/or potassium carbonate, and the product is then etherified with methanol in the presence of an acidic catalyst, without the N-(α-hydroxyethyl)-formamide being separated off beforehand.

## Revendications

1. Procédé de préparation de formamides N-substitués de formule

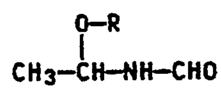

dans laquelle R représente le reste d'un alcanol en $C_1$–$C_8$, par réaction du formamide avec l'acétaldéhyde en présence d'un catalyseur basique pour former le N-(α-hydroxyéthyl)formamide et éthérification ultérieure du N-(α-hydroxyéthyl)formamide avec un alcanol en $C_1$–$C_8$ en présence de catalyseurs acides, caractérisé en ce qu'on mène la réaction du formamide avec l'acétaldéhyde en présence

d'un alcanol en $C_1$–$C_8$, le rapport pondéral du formamide à l'alcanol étant de 1:1 à 15:1.

2. Procédé selon la revendication 1, caractérisé en ce qu'on mène la réaction du formamide avec l'acétaldéhyde en présence d'un alcanol en $C_1$–$C_4$, le rapport pondéral du formamide à l'alcanol en $C_1$–$C_4$ étant de 5:1 à 12:1.

3. Procédé selon les revendications 1 ou 2, caractérisé en ce qu'on mène la réaction du formamide avec l'acétaldéhyde en présence de méthanol.

4. Procédé selon les revendications 1 à 3, caractérisé en ce qu'on fait réagir le formamide avec l'acétaldéhyde en présence de méthanol et de carbonate de sodium et/ou de potassium et on éthérifie ensuite le produit de la réaction, sans préalable du N-(α-hydroxyéthyl)formamide, avec du méthanol en présence de catalyseurs acides.